# EUROPÄISCHE PATENTSCHRIFT

(11) **EP 1 567 077 B1**
(45) Veröffentlichungstag und Bekanntmachung des Hinweises auf die Patenterteilung: **02.01.2008**
(21) Anmeldenummer: 02787686.1
(22) Anmeldetag: 14.11.2002
(51) Int. Cl.: A61B 17/80

(54) **OSTEOSYNTHESEPLATTENSET - 5 VARIANTEN EINES GRUNDTYPS**
OSTEOSYNTHESIS PLATE SET - 5 VARIATIONS OF A BASIC TYPE
CINQ VARIANTES D'UN TYPE DE BASE DE PLAQUES D'OSTEOSYNTHESE

(43) Veröffentlichungstag der Anmeldung: 31.08.2005
(73) Patentinhaber: Ebid, Rainer, 82024 Taufkirchen (DE)
(72) Erfinder: Ebid, Rainer, 82024 Taufkirchen (DE)
(86) Internationale Anmeldenummer: PCT/EP2002/012762
(87) Internationale Veröffentlichungsnummer: WO 2004/045433

(56) Entgegenhaltungen:
- DE-U- 9 014 613
- US-A- 5 690 631
- US-B1- 6 315 852

## Beschreibung

Die Erfindung betrifft eine Osteosyntheseplatte, die in verschiedenen Bereichen unterschiedlich dick ist. Der nächstliegende Stand der Technik ist US-B1-3615852.

Osteosyntheseplatten dienen der Ruhigstellung von Knochen- oder Knochenfragmenten in Relation zueinander. Es zeigt sich, dass im klinischen Alltag mit den bisherigen Methoden noch Probleme bei der Ruhigstellung bestehen.

Die Form eines Dreiecks, die diesen Platten gemein ist, wurde mit dem Hintergrund der Rotationsstabilität auch in Bereichen entwickelt, in denen nicht viel Platz zur Plattenfixation besteht. Die Osteosyntheseplatten sind jedoch nicht an ein spezielles Anwendungsgebiet im Körper des Menschen oder des Tieres gebunden, sondern können dort angewandt werden, wo sie der individuellen Situation entsprechend als sinnvoll erscheinen.

Bei den angegebenen Osteosyntheseplatten handelt es sich um Varianten eines Grundtyps.

Der Grundtyp ist folgendermaßen aufgebaut. An jeder Ecke eines gleichschenkligen Dreiecks befindet sich ein Schraubenloch (A an der Spitze des Dreiecks, B (links) und C (rechts) jeweils am Ende eines der gleichlangen Schenkel). In der Mitte zwischen beiden Schenkeln zieht zur Stabilisierung ein weiterer Steg, der das gleichschenklige Dreieck in zwei äquivalente rechtwinklige Dreiecke teilt. In diesem Steg befindet sich ein weiteres Schraubenloch (D) auf halbem Wege von der Spitze des Dreiecks (A) zum gegenüberliegenden Schenkel. Am Kreuzungspunkt des Steges mit dem Schenkel befindet sich nochmals ein Schraubenloch (E). Die Abstände von A zu D, von D zu E, von B zu E und C zu E sind alle jeweils gleich groß. Dies ist die Grundform, die allen 5 Varianten des Osteosyntheseplattensets gemein ist. Bei einer Variante sind sowohl der Steg als auch die beiden Schenkel verlängert. An jedem der 3 Enden ist wiederum ein Schraubenloch (F in Verlängerung des Steges, G (links) und H (rechts) jeweils in Verlängerung eines der gleich langen Schenkel). Diese 3 Schraubenlöcher liegen auf einer Linie, die parallel zum dritten Schenkel ist (auf dem B, E und C liegen). Der Abstand von E zu F ist genauso groß, wie von A zu D.

Ausgehend von dieser Platte fehlen bei den weiteren Varianten die folgenden Schraubenlöcher:

Bei 1 fehlen G und H, 2 stellt die eben beschriebene Variante dar, bei 3 fehlt F, bei 4 fehlt H und spiegelbildlich dazu fehlt bei 5 G. Zum besseren Verständnis sind die Osteosyntheseplatten in dieser Reihenfolge als Zeichnungen dargestellt. Die Aufsicht auf die Platten zeigt dabei die Plattenform inklusive der runden Schraubenlöcher. Jedes der Schraubenlöcher kann aber auch oval sein oder irgendeine eckige Form haben.

Jede der Ecken jeder einzelnen Platte kann eckig oder abgerundet sein. Jeder der Schenkel und/oder der Steg können im Bereich der Schraubenlöcher verbreitert sein, um genügend Plattenmaterial rund um jedes der Schraubenlöcher zu haben. Auf den Zeichnungen sind Platten mit Verbreiterungen im Bereich der Schraubenlöcher zu sehen.

Die Größe jeder der 5 Varianten des Osteosyntheseplattensets kann variieren.

Die Dicke jeder einzelnen der 5 Varianten des Osteosyntheseplattensets kann in allen Bereichen ein und derselben Platte gleich oder in den verschiedenen Bereichen ein und derselben Platte unterschiedlich sein.

Die 5 Varianten des Osteosyntheseplattensets sind auf keine Region im Körper des Menschen oder Tieres beschränkt. Eine konkrete Anwendungsmöglichkeit findet sich aber beispielsweise bei Collum mandibulae - Frakturen des Menschen (Das Collum Mandibulae ist ein Teil des Unterkiefers.).

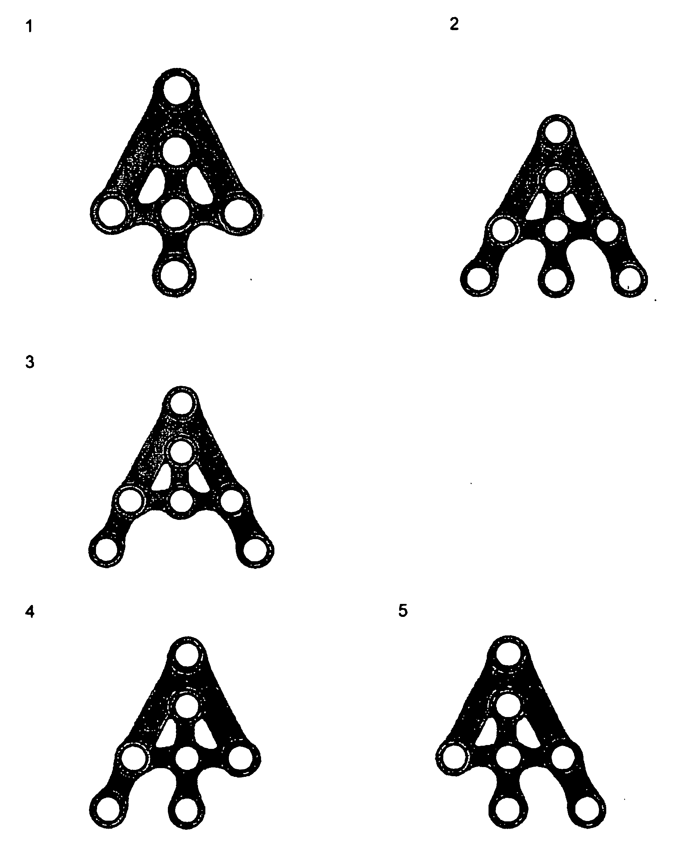

## Patentansprüche

1. Osteosyntheseplatte **dadurch gekennzeichnet, daß** die Osteosyntheseplatte in der Form eines gleichschenkligen Dreiecks ABC ist,
wobei durch einen Mittelsteg AE das Dreieck in zwei äquivalente Dreiecke ABE und ACE geteilt wird und Punkt D derart auf dem Mittelsteg AE liegt, daß AD und DE, ebenso DE, BE und CE, äquidistant sind,
wobei Schraubenlöcher sich an den Punkten A, B, C, D, E befinden,
wobei der Steg AB und der Steg AC mit der seitlichen Begrenzung des Schraubenloches D verschmolzen sind,
wobei der Steg AB gleichzeitig die Schraubenlöcher von A und B sowie D und B direkt verbindet und der Steg AC gleichzeitig die Schraubenlöcher von A und C sowie D und C direkt verbindet,
wobei der Steg AD vollständig mit den angrenzenden Teilen der Stege AB und AC verschmolzen ist und A und D somit flächig verbunden sind,
wobei der Schenkel AB zu ABG bzw. AC zu ACH und der Mittelsteg AE zu AEF mit EF äquidistant zu DE und BEC parallel zur gedachten Linie GFH verlängert sind,
wobei an den Punkten F, G, und H Schraubenlöcher sind,
wobei vier weitere Varianten der Osteosyntheseplatte verfügbar sind, wobei nur der Punkt F oder der Punkt G oder der Punkt H fehlt oder die Punkte G und H gleichzeitig fehlen, jeweils inklusive der Verbindung zum Nachbarloch.

## Claims

1. Osteosynthesis plate, **characterised by** an osteosynthesis plate in
the form of an ABC isosceles triangle,
whereby the triangle is divided into two equivalent triangles, ABE and ACE, by means of a central bar AE, and where point D lies on the central bar AE in such a way that AD and DE and DE, BE and CE are equidistant,
whereby screw holes are located at points A, B, C, D and E,
whereby bar AB and bar AC are fused with the lateral limit of
screw hole D,
whereby bar AB simultaneously directly connects screw holes A and B and D and B, and bar AC simultaneously directly connects screw holes A and C and D
and C,
whereby bar AD is completely fused with the adjoining parts of bars AB and
AC, with the result that A and D establish a planar connection,
whereby sides AB to ABG, AC to ACH and the central bar AE to AEF, where EF is equidistant to DE and BEC is parallel to the intended line GFH, are elongated,
whereby at points F, G and H screw holes are located,
whereby four further versions of the osteosynthesis plate are available, whereby only point F, or point G, or point H are missing, or the points G and H are simultaneously missing, in each case the connection to the neighbouring hole included.

## Revendications

1. Plaque d'ostéosynthèse **caractérisée en ce que** ladite plaque d'ostéosynthèse prend la forme d'un triangle isocèle ABC,
une nervure médiane AE divisant le triangle en deux triangles équivalents ABE et ACE et le point D étant situé sur la nervure médiane de sorte que AD et DE d'une part et DE, BE et CE d'autre part sont équidistants,
des trous de vis se trouvant aux points A, B, C, D et E,
la nervure AB et la nervure AC étant confondues avec la bordure latérale du trou de vis D,
la nervure AB reliant simultanément et les trous de vis A et B et les trous de vis D et B et la nervure AC reliant simultanément et les trous de vis A et C et les trous de vis D et C,
la nervure AD étant entièrement confondue avec les parties avoisinantes des nervures AB et AC, si bien que A et D sont reliés en surface,
le côté AB étant prolongé pour former ABG, le côté AC étant prolongé pour former ACH et la nervure médiane AE étant prolongée pour former AEF, EF étant équidistant par rapport à DE et BEC étant parallèle à la ligne GFH, des trous de vis se trouvant aux points F, G et H,
quatre autres variantes de la plaque d'ostéosynthèse étant disponibles,
seul le point F ou le point G ou le point H étant supprimé avec son lien par rapport au trou voisin ou bien les points G et H étant supprimés tous les deux avec leur lien par rapport au trou voisin.
